# EUROPEAN PATENT SPECIFICATION

(11) **EP 2 535 022 B1**
(45) Date of publication and mention of the grant of the patent: **07.10.2015**
(21) Application number: 11169886.6
(22) Date of filing: 14.06.2011
(51) Int. Cl.: A61F 2/44, A61F 2/46

(54) **Device for inserting an intervertebral implant into a body and system comprising an intervertebral implant and a device for inserting the same**
Vorrichtung zum Einsetzen eines Bandscheibenimplantats in einen Körper und System mit Bandscheibenimplantat und Vorrichtung zum Einsetzen davon
Dispositif pour insérer un implant intervertébral dans un corps et système comprenant un implant intervertébral et un dispositif pour l'insérer

(43) Date of publication of application: 19.12.2012
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE)
(74) Representative: Prüfer & Partner GbR European Patent Attorneys

(56) References cited:
- US-A1- 2005 038 431
- US-A1- 2005 080 422
- US-A1- 2007 162 129
- US-A1- 2007 213 826
- US-A1- 2008 221 694
- US-A1- 2010 114 105
- US-A1- 2010 256 759

## Description

The invention relates to a device for inserting an intervertebral implant into a body and to a system comprising an intervertebral implant and a device for inserting the same. The intervertebral implant has a top surface configured to engage a first vertebral body, a bottom surface configured to engage a second vertebral body and a side wall connecting the top surface and the bottom surface. In the side wall, an elongate opening is provided. The device for inserting the intervertebral implant comprises a flexible end portion that can assume a first state in which it can be introduced into and removed through the opening and a second state in which it cannot be removed. In the second state the device is connected to the intervertebral implant. The implant and the device for inserting the implant are movable relative to each other along the elongate opening.

A device for the insertion of surgical implants is known from US 7,235,082 B2. The device comprises a shaft defining a conduit and having proximal and a distal end. At least one movable element is provided that includes a leg extending through the conduit and a foot. The movable element is movable relative to the shaft between a first position, wherein the foot extends beyond the distal end and a second position, wherein the foot is substantially adjacent to the distal end and wherein the distal end extends through an opening of the implant.

An intervertebral implant configured to engage with an insertion device for inserting between first and second vertebral members is known from US 2007/0162129 A1. The intervertebral implant has an opening including an elongated shape that extends through the side wall. A connection member including a receptacle is contained within the side wall. The insertion device has a first end that may be configured to connect with the connection member of the implant body. The first end may be selectively positionable between orientations to provide for the connection.

Document US 2010/0256759 A1 discloses an insertion instrument for intervertebral spacers having a top surface, a bottom surface and a sidewall connecting the top surface and the bottom surface. The wall comprises an elongated opening in the sidewall. The instrument has central shaft with a threaded tip portion for engaging an insert of the spacer and a forked free end with a pair of tongs for engaging the spacers wherein the movement for engaging is limited. Depending on the engagement of the forked free end and the spacer, the spacer is movable around the insert of the spacer.

Document US 2008/0221694 A1 discloses a spacer system with an interbody spacer and an insertion tool. The spacer has an elongated opening in a sidewall between a top surface and a bottom surface and an expandable tip of the insertion tool can enter the elongated opening. The expandable can expand such that it rotatably secures the space.

It is the object of the invention to provide a device for inserting an intervertebral implant and a system comprising an intervertebral implant and a device for inserting the same that is simplified in terms of its use and and that is more flexible in view of the final positioning of the implant.

The object is solved by a device according to claim 1 and by a system according to claim 12. Further developments are given in the dependent claims.

The device for insertion an intervertebral implant simplifies the procedure of insertion since the connection between the device and the implant can be easily fixed and loosened. When the connection is loose, the device can be moved into a desired position by rotating the implant relative to the device in a plane that extends through the center of the side wall in a vertical direction. The design of the device for inserting the implant ensures that a maximum of the hollow interior space of the implant can be used for fusion.

When the intervertebral implant is rotated to achieve its position between the vertebrae it is safely held by the device and protected against disconnection from the device.

The final positioning the implant and the removal of the device is simplified.

An existing intervertebral implant can be modified to be adapted to the insertion device.

Further features and advantages of the invention will become apparent from the description of the accompanying drawings. In the drawings:
- Fig. 1: shows a perspective view of an embodiment of a system consisting of an intervertebral implant and a device for inserting the intervertebral implant.
- Fig. 2: shows an exploded perspective view of the system of Fig. 1 wherein a portion of the device for insertion is depicted.
- Fig. 3a): shows a cross-sectional view of the intervertebral implant and an end portion of the device for insertion, wherein a cross-section is taken along line C-C in Fig. 3b).
- Fig. 3b): shows a top view of the system consisting of the intervertebral implant and portion of the device as shown in Fig. 3a.
- Fig. 4a): shows a cross-sectional view of the intervertebral implant and the device for inserting, wherein the device for inserting is in a first state that allows introduction to the opening of the implant.
- Fig. 4b): shows a top view of the system shown in Fig. 4a).
- Fig. 5a): shows a cross-sectional view of the intervertebral implant and the device for inserting introduced into the opening and wherein a pin of the device is in a retracted position.
- Fig. 5b): shows a top view of the intervertebral implant and the device for inserting of Fig. 5a).
- Fig. 6a): shows a cross-sectional view of the intervertebral implant and the device for inserting introduced into the opening, wherein the pin of the device is in a protruding position.
- Fig. 6b): shows the intervertebral implant and the device for insertion of Fig. 6a) in a top view.
- Fig. 7: shows the intervertebral implant and the device for inserting inserted into the opening of the intervertebral implant, wherein the intervertebral implant is rotated relative to the device for inserting in a plane extending through the center of the side wall of the implant in a vertical direction.
- Fig. 8: shows the intervertebral implant and the device for insertion removed from the intervertebral implant.
- Figs. 9a) to 9f): show steps for inserting and positioning the intervertebral implant into a body.

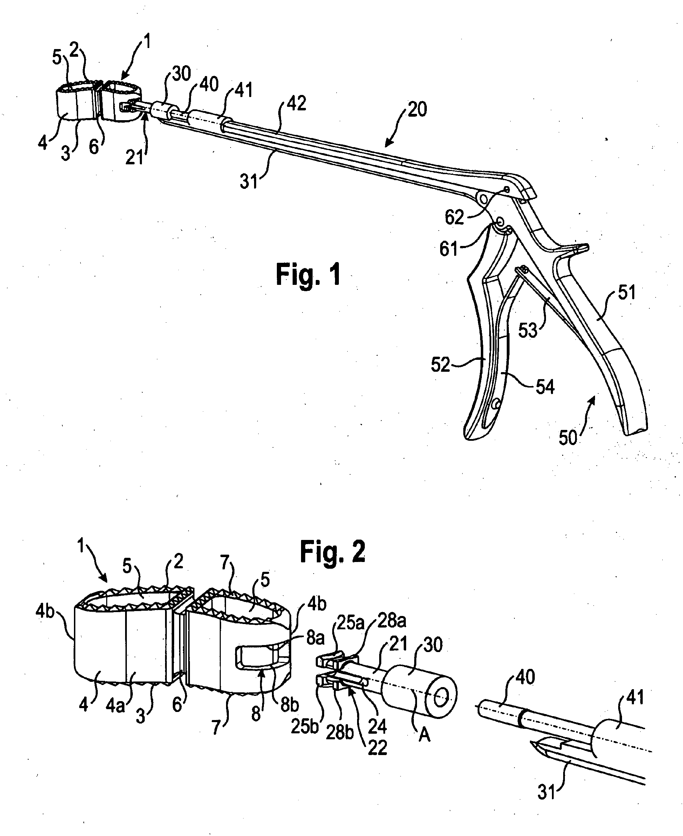

Fig. 1 shows a perspective view of one embodiment of an intervertebral implant 1 and a device 20 for inserting the intervertebral implant 1 that is attached to the implant. As shown in particular in Figs. 1 and 2, the intervertebral implant 1 includes a top face 2 and a bottom face 3 that are configured to engage respective end plates of a first vertebral body and a neighbouring second vertebral body. The top face 2 and the bottom face 3 are connected via a side wall 4 that defines an interior hollow section 5. Usually, the top face 2 and the bottom face 3 have openings so that the hollow interior section 5 extends into the top face 2 and the bottom face 3. In the embodiment shown, the top face 2 and the bottom face 3 are formed by the upper and lower rim of the side wall 4. Furthermore, a center wall 6 may be provided that separates the hollow interior section 5 in two parts. The height of the side wall 4 is sized so as to allow insertion between a first and a second vertebral body. The height may be larger at the center wall 6 and may decrease towards the outer ends. Teeth 7 or other engagement portions are provided that project from the top face 2 and the bottom face 3 for engaging the end plates.

In the embodiment shown, the implant 1 has two opposing long sides 4a and two opposing short sides 4b connecting the long sides. The short sides 4b are rounded. The contour of side wall 4 may be arcuate, for example it may have a kidney-shape or banana-shape.

As shown in particular in Fig. 2, an opening 8 in the side wall 4 is provided that extends completely through the side wall 4 into the interior hollow section 5. The opening 8 has an elongate shape and extends preferably over a length in a circumferential direction along a short side 4b. The opening 8 may have a substantially rectangular contour with an upper edge 8a and a lower edge 8b and is located substantially in the middle of the side wall in a vertical direction between the top face and the bottom face.

The implant shown in the embodiment is only an example. The contour and shape of the implant may be different according to the specific clinical requirements. For example, the contour may have any other shape, for example, it may be circular or rectangular, oval etc. The height of the side wall 4 may be constant throughout the implant. The opening can be at another position. Only one opening or more than two openings may be provided. Also, it is possible to adapt existing intervertebral implants without such an opening by providing it with an opening. Further, the center wall can be omitted.

An embodiment of the device for inserting the intervertebral implant will now be explained with reference to Figs. 1 to 8. The device for inserting 20 comprises an end portion 21 that is configured to be introduced into the opening 8 of the intervertebral implant 1 and to hold the intervertebral implant 1. The end portion 21 is a sleeve-like member having a flexible front portion 22. The flexible front portion 22 is formed by two opposing tongues 23a, 23b that are generated by a slot 24 extending from the free edge of the sleeve-like end portion 21 through opposing portions of the sleeve.

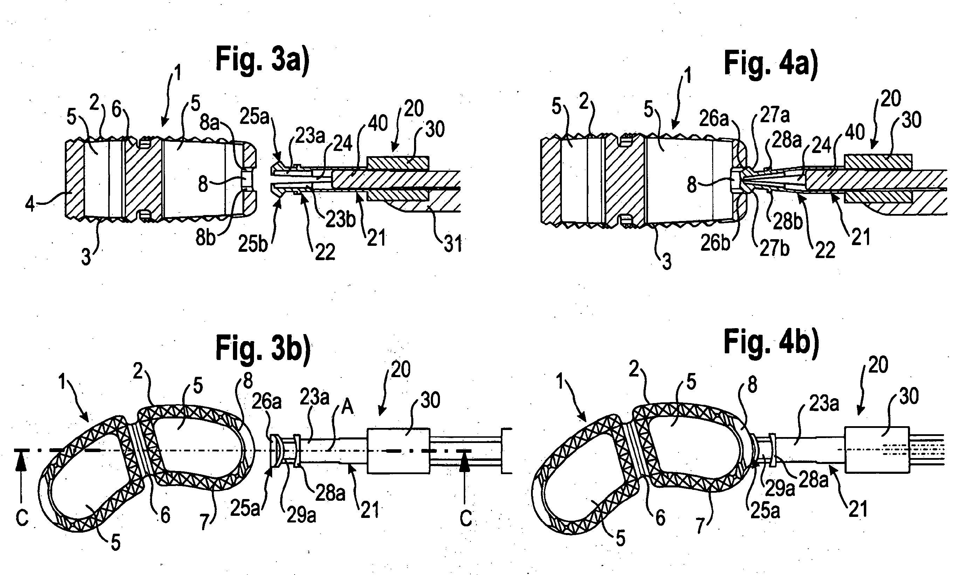

Each tongue 23a, 23b comprises at its free end a thickened portion 25a, 25b.The thickened portions 25a, 25b extend outward so that the outer diameter of the tongues is increased thereby, as can be seen, for example, in Figs. 3a) and 3b). The contour of the thickened portions 25a, 25b seen in a direction perpendicular to the sleeve axis A is substantially rectangular. The thickened portions 25a, 25b each have a chamfered front wall 26a, 26b and a chamfered rear wall 27a, 27b. The rear wall 27a, 27b may be curved with a curvature corresponding to the curvature of the side wall 4 in the region of the opening 8.

In addition, each tongue 23a, 23b has an outwardly extending rib 28a, 28b at a distance from the thickened portion 25a, 25b that is equal or greater than the wall thickness of the side wall 4 of the implant in the opening 8. Hence, the wall around the opening 8 of the intervertebral implant 1 fits into the portion 29a,29b. In a top view, as shown in Fig. 3a) and 4a), the side of the rib 28a, 28b that faces the free end of the sleeve-like end portion 21 is inwardly curved. The curvature may correspond to the curvature of the side wall 4 in the region of the opening 8. The portions 29a, 29b between the thickened portions 25a, 25b and the ribs 28a, 28b are flat to allow an even contact with the upper and lower edge 8a, 8b of the opening, respectively.

The outer diameter of the flexible front portion 22 of the sleeve-like end portion 21 is sized such that in a non-compressed state of the tongues 23a, 23b the thickened portions 25a, 25b have a maximum outer diameter that is greater than the height of the opening 8. Also, when the tongues 23a, 23b are non-compressed, the outer diameter of the portions 28a, 28b is slightly larger than the height of the opening. Simultaneously, the outer diameter of the sleeve at the portions 29a, 29b is smaller than the height of the opening 8. The length and width and other sizes of the slot 24 are such that it allows compression of the tongues 23a, 23b towards each other and introduction of the flexible front portion 22 into the opening 8 of the intervertebral implant. The outer diameter of the sleeve-like end portion 21 at the position of the slot 24 is smaller than the length of the elongate opening 8 so that the flexible front portion 22 may move along the length of the elongate opening 8 in a circumferential direction.

The sleeve-like end portion 21 has a rear portion 30 with a larger outer diameter. The rear portion 30 is connected to a first grip portion 51 of a tongs-like handle 50 that comprises the first grip portion 51 and a second grip portion 52.

The device for inserting 20 further comprises a pin 40 extending into the sleeve-like end portion 21. The pin 40 has an outer diameter that allows it to slide within the sleeve-like end portion 21. The inner diameter of the flexible front portion 22 of the sleeve-like end portion 21 is slightly smaller than the outer diameter of the pin 40 so that, when the pin 40 is moved between the tongues 23a, 23b, it slightly spreads the tongues 23a, 23b apart from each other. Thus, the sleeve-like end portion 21 and the pin 40 cooperate in the manner of a collet chuck with pin. The pin 40 is mounted to a portion 41 with an outer diameter greater than that of the pin that can slide in a connection bar 31 connecting the sleeve-like end portion 21 with the grip portion 51. The pin 40 is thereby connected through a connection bar 42 with the other grip portion 52 of the handle 50. The grip portions 51, 52 are connected via hinges 61, 62 to the connection bars 31, 42, respectively, so that, like with tongs, a moving of the grip portions 51 52 towards each other moves the pin 40 relative to the sleeve-like end portion 21.

The pin 40 can assume a first position in which it is retracted from the tongues 23a, 23b, as shown, for example in Figs. 3 to 4. The pin further can assume a second position in which it protrudes into the sleeve-like end portion 21 between the tongues 23a, 23b and spreads them apart.

The grip portions 51, 52 are biased away from each other via leaf springs 53, 54. Due to the hinges 61, 62 the handle 50 is arranged at an angle with respect to the connecting bars 31, 42. This facilitates handling and increases the possibilities for bringing the implant to its final position.

The connection of the pin 40 and the sleeve-like end portion 21 to the handle 50 is only exemplary. Other constructions and other types of handles can be used to allow the relative movement of the pin with respect to the sleeve-like end portion as described above.

The implant 1 as well as the portions of the device for inserting the implant 20 that are in contact with a patient's body are made of a biocompatible material. For example, the implant and/or the device for inserting are made of stainless steel or titanium or biocompatible metal alloy, such as nickel-titanium alloys or made of a biocompatible plastic material, such as for example PEEK (polyetheretherketone).

The functioning of the device for inserting the implant will now be explained with reference to Figs. 3 to 9. The device for insertion and the implant are oriented relative to each other such that the slot is parallel to the upper or lower edge of the elongate opening. Hence, the tongues are on top of each other seen in a height direction of the implant. First, as shown in Figs. 3a) and 3b) the pin 40 is in the first position that is the retracted position. The tongues 23a, 23b are configured to be flexibly pressed towards each other as shown in Fig. 4a. When the tongues 23a, 23b are pressed towards each other, the flexible front portion 22 can be introduced into the elongate opening 8 of the implant 1. During introduction, the chamfered front wall 26a, 26b of the tongues 23a, 23b slides along the upper and lower edge 8a, 8b of the elongate opening until the uppermost and lowermost portion of the thickened portions 25a, 25b are in contact with the upper and lower edge 8a, 8b of the elongate opening 8. This maintains the tongues 23a, 23b in a compressed state during introduction. The tongues may be slightly pre-compressed by bending them towards each other in the retracted position of the pin 40.

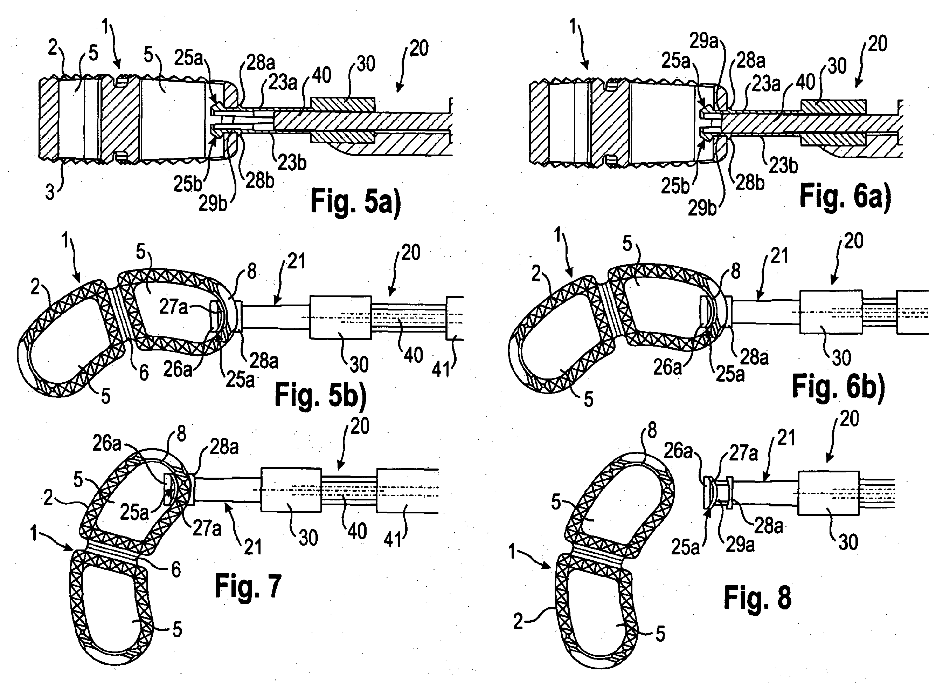

When the thickened portions 25a, 25b of the flexible front portion 22 have passed the elongate opening 8 and entered the hollow interior section 5 of the implant 1, the tongues 23a, 23b spread apart from each other until the upper and lower edge 8a, 8b of the elongate opening rests in the space portion 29a, 29b as can be seen in Figs. 5 and 6. In this condition, the tongues 23a 23b can still be compressed towards each other. This makes it possible that the flexible front portion 22 is still movable in the elongate opening 8 in the lengthwise direction of the opening. Hence, the flexible front portion 22 can be moved into a suitable position for introducing the implant 1 into the surgical side. The ribs 28a, 28b act as a stop that prevents further introduction of the device into the implant.

As soon as the implant and the device for inserting the implant are positioned correctly with respect to each other, the pin 40 is pushed further into the flexible front portion 22 thereby spreading the tongues 23a, 23b slightly apart from each other until they abut against the upper and lower edge 8a, 8b of the elongate opening 8, respectively. Thereby, the implant is firmly held through engagement of the upper and lower edge 8a, 8b of the opening with the portions 29a, 29b of the sleeve-like end portion 21. This is shown in particular in Figs. 6a and 6d.

Finally, after the implant has been positioned at the implantation site, the pin 40 is moved back from its second position into the retracted position and the tongues 23a, 23b are drawn out from the opening. Due to the chamfered rear wall 27a, 27b it is possible to press the tongues 23a, 23b together when the flexible front portion 22 is drawn out from the implant. This allows retracting the flexible front portion 22 through the elongate opening.

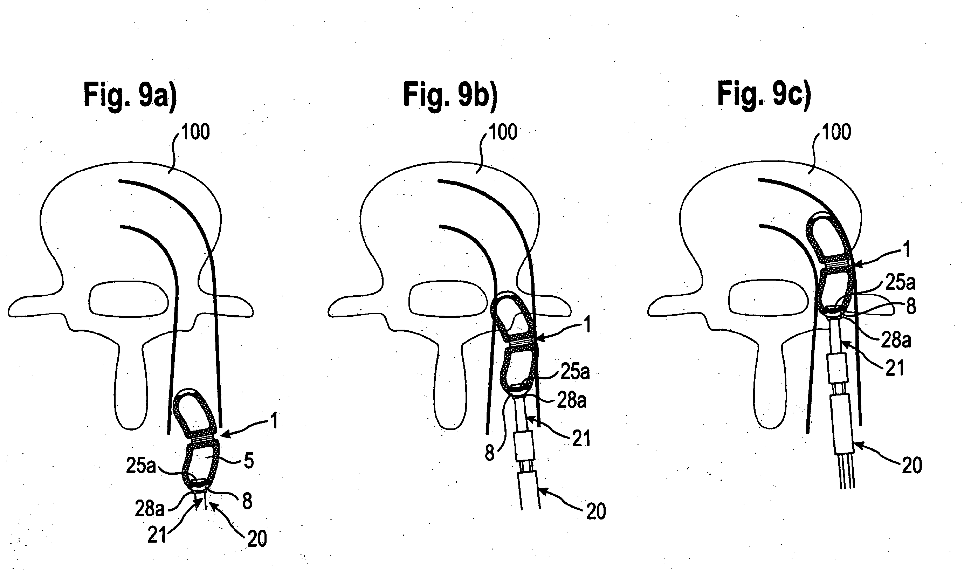

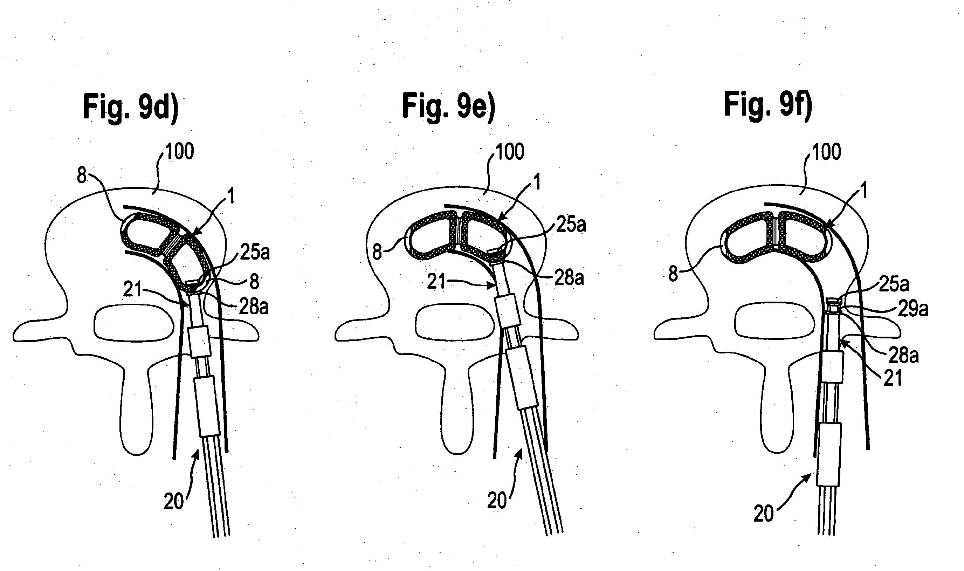

The use of the implant during surgery will now be described with respect to Figs. 9a) to 9f). Figs. 9a) to 9f) show steps for inserting and positioning the intervertebral implant into a body. First, as shown in Fig. 9a) the device for inserting the implant 20 is connected to the implant 1 preferably in such a way that the flexible front portion 22 is positioned at approximately the center of the elongate opening 8 in a lengthwise direction. By moving the pin 40 into the second position, the connection between the implant and the device for inserting is fixed. Then the implant is is introduced into the intervertebral space between two neighbouring vertebrae, one of them is shown as vertebra 100 in the drawings. The narrow side 4b of the implant opposite to the device 20 is the leading side. In the method shown, the intervertebral implant 1 is introduced in the space between the vertebral bodies using a posterior and lateral approach to access the space between the vertebral bodies.

As soon as the implant 1 and the device for insertion 20 experience resistance and cannot be pushed further as shown in Fig. 9c), the fixation between the implant and the device for inserting 20 is loosened in that the pin is retracted into the first position. Hence, the implant and the device are movable relative to each other, because the sleeve-like end portion 21 can move in the elongate opening 8. By means of this, the angle between the implant and the device for inserting can be changed. As shown in Figs. 9c) to 9e), the implant is then pushed into its end position between the vertebral bodies.

Thereafter, the device for insertion is removed from the implant by drawing the flexible front portion 22 out of the opening 8.
Since the device for inserting is connected to and separated from the implant in an easy manner, the handling is simplified. Due to the thickened portions and the ribs that act as stops, a removal of the device during the process of inserting the implant is prevented.

Modifications of the device for inserting the implant are possible. For example, the number of flexible tongues may vary. Also the shape of the tongues can vary. Instead of the pin another spreading member can be used.

## Claims

1. Device for inserting (20) an intervertebral implant (1) into a body, wherein the intervertebral implant has a top surface (2) configured to engage a first vertebral body, a bottom surface (3) configured to engage a second vertebral body and a side wall (4) connecting the top surface (5) and the bottom surface (3) and an elongate opening (8) in the sidewall (4), the elongate opening (8) having a height and a length greater than the height the device (20) comprising
a flexible end portion (21) having a free end and having a diameter smaller than the length of the elongate opening (8), wherein the flexible end portion (21) can assume a first state wherein it is configured to be introduced into the opening (8) and a second state wherein it is configured to engage the implant to provide a connection between the implant (1) and the device (20), and wherein the flexible end portion comprises a stop (28a, 28b) at a distance from the free end that limits the insertion of the flexible end portion (21) into the implant through the elongate opening, **characterised in that**, the flexible end portion (21) comprises an outward protrusion (25a, 25b) at its free end and an outward protrusion (28a, 28b) at a distance from its free end, the distance being greater than the wall thickness around the elongate opening (8) so that the implant (1) is held by the device (20) in the second state.

2. The device of claim 1, wherein the flexible end portion (21) is removable through the opening (8) in the first state.

3. The device of claim 1 or 2, wherein the outward protrusions are extending in a direction of the height of the elongate opening (8)

4. The device of one of claims 1 to 3, wherein the flexible end portion (21) comprises at least two tongues (23a, 23b) separated by at least one slot (24).

5. The device of one of claims 1 to 4, wherein the flexible end portion (21) has a chamfered surface portion (26a, 26b) at its free end that is configured to allow sliding along an edge (8a, 8b) of the elongate opening (8).

6. The device of one of claims 1 to 5, wherein the flexible end portion (21) has a chamfered rear surface portion (27a, 27b) facing away from the free end and configured to allow sliding along an edge (8a, 8b) of the elongate opening (8).

7. The device of one of claims 1 to 6, wherein a spreading member is slidably arranged in the flexible end portion (21).

8. The device of claim 7, wherein the spreading member is a pin (40) that can assume a first retracted position in which the flexible end portion (21) is configured to be compressible and extendable and a second protruding position in which it protrudes into the flexible end portion (21) thereby preventing the flexible end portion from being compressed.

9. The device of one of claims 1 to 8, wherein the stop (28a, 28b) has a curved shape adapted to a curved portion of the side wall (4) of the implant (1) around the elongate opening (8).

10. The device of one of claims 6 to 9, wherein the flexible end portion (21) and the spreading member each are connected to a grip portion (51, 52) of a handle (50).

11. The device of claim 10, wherein the handle (50) is configured to move the spreading member relative to the flexible end portion (21).

12. A system comprising an intervertebral implant (1) and a device for inserting (20) the intervertebral implant into a body, wherein the intervertebral implant (1) has a top surface (2) configured to engage a first vertebral body, a bottom surface (3) configured to engage a second vertebral body and a side wall (4) connecting the top surface (5) and the bottom surface (3) and an elongate opening (8) in the sidewall (4),
and wherein the device for inserting (20) is a device according to one of claims 1 to 11.

13. The system of claim 12, wherein the elongate opening (8) has an arcuate shape in the lengthwise direction and wherein the flexible end portion (21) has an arcuate portion (25a, 25b; 28a, 28b) adapted to the arcuate shape of the opening (8).

14. A method for coupling an intervertebral implant (1) to a device for inserting (20) the intervertebral implant (1) wherein the intervertebral implant has a top surface (2) configured to engage a first vertebral body, a bottom surface (3) configured to engage a second vertebral body and a side wall (4) connecting the top surface (5) and the bottom surface (3) and an elongate opening (8) in the sidewall (4), the opening having a height and a length greater than the height,
and wherein the device for inserting (20) is a device according to one of claims 1 to 11, the method comprising a step of
introducing the flexible end portion (21) into the opening (8) by compressing it in the height direction of the opening and connecting the implant (1) to the device for inserting (20) by spreading the flexible end portion (21) in the height direction.

15. The method of claim 14, further comprising a step of guiding the device for inserting (20) along the elongate opening (8) in a length direction.

## Patentansprüche

1. Vorrichtung zum Einsetzen (20) eines Zwischenwirbelimplantats (1) in einen Körper, wobei das Zwischenwirbelimplantat eine obere Fläche (2), die ausgebildet ist, in einen ersten Wirbelkörper einzugreifen, eine untere Fläche (3), die ausgebildet ist, in einen zweiten Wirbelkörper einzugreifen, und eine Seitenwand (4), die die obere Fläche (5) und die untere Fläche (3) verbindet, und eine langgestreckte Öffnung (8) in der Seitenwand (4) hat, wobei die langgestreckte Öffnung (8) eine Höhe und eine Länge hat, die größer als die Höhe ist, wobei die Vorrichtung (20)
einen flexiblen Endabschnitt (21) aufweist, der ein freies Ende und einen Durchmesser hat, der kleiner als die Länge der langgestreckten Öffnung (8) ist, wobei der flexible Endabschnitt (21) einen ersten Zustand, wobei er ausgebildet ist in die Öffnung (8) eingeführt zu werden, und einen zweiten Zustand annehmen kann, wobei er ausgebildet ist, in das Implantat einzugreifen, um eine Verbindung zwischen dem Implantat (1) und der Vorrichtung (20) bereitzustellen, und wobei der flexible Endabschnitt in einem Abstand von dem freien Ende einen Anschlag (28a, 28b) aufweist, der das Einführen des flexiblen Endabschnitts (21) durch die langgestreckte Öffnung in das Implantat begrenzt,
**dadurch gekennzeichnet, dass**
der flexible Endabschnitt (21) an seinem freien Ende einen nach außen gerichteten Vorsprung (25a, 25b) und in einem Abstand von dem freien Ende einen nach außen gerichteten Vorsprung (28a, 28b) aufweist, wobei der Abstand größer als die Wanddicke um die langgestreckte Öffnung (8) ist, so dass das Implantat (1) in dem zweiten Zustand durch die Vorrichtung (20) gehalten wird.

2. Vorrichtung von Anspruch 1, wobei der flexible Endabschnitt (21) in dem ersten Zustand durch die Öffnung (8) entfernbar ist.

3. Vorrichtung von Anspruch 1 oder 2, wobei sich die nach außen gerichteten Vorsprünge in einer Richtung der Höhe der langgestreckten Öffnung (8) erstrecken.

4. Vorrichtung von einem der Ansprüche 1 bis 3, wobei der flexible Endabschnitt (21) zumindest zwei Zungen (23a, 23b) aufweist, die durch zumindest einen Schlitz (24) getrennt sind.

5. Vorrichtung von einem der Ansprüche 1 bis 4, wobei der flexible Endabschnitt (21) an seinem freien Ende einen abgeschrägten Flächenabschnitt (26a, 26b) hat, der ausgebildet ist, ein Gleiten entlang einer Kante (8a, 8b) der langgestreckten Öffnung (8) zu ermöglichen.

6. Vorrichtung von einem der Ansprüche 1 bis 5, wobei der flexible Endabschnitt (21) einen abgeschrägten hinteren Flächenabschnitt (27a, 27b) hat, der von dem freien Ende weggewandet ist und ausgebildet ist, ein Gleiten entlang einer Kante (8a, 8b) der langgestreckten Öffnung (8) zu ermöglichen.

7. Vorrichtung von einem der Ansprüche 1 bis 6, wobei ein Spreizelement in dem flexiblen Endabschnitt (21) verschiebbar angeordnet ist.

8. Vorrichtung von Anspruch 7, wobei das Spreizelement ein Stift (40) ist, der eine erste zurückgezogene Position, in der der flexible Endabschnitt (21) ausgebildet ist, zusammendrückbar und ausdehnbar zu sein, und eine zweite vorstehende Position einnehmen kann, in der er in den flexiblen Endabschnitt (21) vorsteht und es dabei verhindert, dass der flexible Endabschnitt zusammengedrückt werden kann.

9. Vorrichtung von einem der Ansprüche 1 bis 8, wobei der Anschlag (28a, 28b) eine gekrümmte Form hat, die an einen gekrümmten Abschnitt der Seitenwand (43) des Implantats (1) um die langgestreckte Öffnung (8) angepasst ist.

10. Vorrichtung von einem der Ansprüche 6 bis 9, wobei sowohl der flexible Endabschnitt (21) als auch das Spreizelement mit einem Griffabschnitt (51, 52) eines Handgriffs (50) verbunden sind.

11. Vorrichtung von Anspruch 10, wobei der Griff (50) ausgebildet ist, das Spreizelement relativ zu dem flexiblen Endabschnitt (21) zu bewegen.

12. System, das ein Zwischenwirbelimplantat (1) und eine Vorrichtung zum Einsetzen (20) des Zwischenwirbelimplantats in einen Körper aufweist, wobei das Zwischenwirbelimplantat (1) eine obere Fläche (2), die ausgebildet ist, in einen ersten Wirbelkörper einzugreifen, eine untere Fläche (3), die ausgebildet ist, in einen zweiten Wirbelkörper einzugreifen, und eine Seitenwand (4), die die obere Fläche (5) und die einen untere Fläche (3) verbindet, und eine langgestreckte Öffnung (8) in der Seitenwand (4) aufweist,
und wobei die Vorrichtung zum Einsetzten (20) eine Vorrichtung gemäß einem der Ansprüche 1 bis 11 ist.

13. System von Anspruch 12, wobei die langgestreckte Öffnung (8) eine in der Längsrichtung gekrümmte Form hat, und wobei der flexible Endabschnitt (21) einen gekrümmten Abschnitt (25a, 25b; 28a, 28b) hat, der an die gekrümmte Form der Öffnung (8) angepasst ist.

14. Verfahren zum Koppeln eines Zwischenwirbelimplantats (1) an eine Vorrichtung zum Einsetzen (20) des Zwischenwirbelimplantats (1), wobei das Zwischenwirbelimplantat eine obere Fläche (2), die ausgebildet ist, in einen ersten Wirbelkörper einzugreifen, eine untere Fläche (3), die ausgebildet ist, in einen zweiten Wirbelkörper einzugreifen, und eine Seitenwand (4), die die obere Fläche (5) und die untere Fläche (3) verbindet, und eine langgestreckte Öffnung (8) in der Seitenwand (4) hat, wobei die Öffnung eine Höhe und eine Länge hat, die größer als die Höhe ist,
und wobei die Vorrichtung zum Einsetzten (20) eine Vorrichtung gemäß einem der Ansprüche 1 bis 11 ist,
wobei das Verfahren einen Schritt eines Einführens des flexiblen Endabschnitts (21) in die Öffnung (8), indem es in der Höhenrichtung der Öffnung zusammengedrückt wird, und eines Verbindens des Implantats (1) mit der Vorrichtung zum Einsetzen (20) durch Spreizen des flexiblen Endabschnitts (21) in der Höhenrichtung, aufweist.

15. Verfahren von Anspruch 14, ferner einen Schritt eines Führens der Vorrichtung zum Einsetzen (20) entlang der langgestreckten Öffnung (8) in einer Längsrichtung.

## Revendications

1. Dispositif d'insertion (20) d'un implant intervertébral (1) dans un corps, où l'implant intervertébral a une surface supérieure (2) configurée pour s'engager avec un premier corps vertébral, une surface inférieure (3) configurée pour s'engager avec un deuxième corps vertébral et une paroi latérale (4) reliant la surface supérieure (5) et la surface inférieure (3) et une ouverture allongée (8) dans la paroi latérale (4), l'ouverture allongée (8) ayant une hauteur et une longueur supérieure à la hauteur,
le dispositif (20) comprenant une partie d'extrémité flexible (21) ayant une extrémité libre et ayant un diamètre inférieur à la longueur de l'ouverture allongée (8), où la partie d'extrémité flexible (21) peut adopter un premier état où elle est configurée pour être introduite dans l'ouverture (8) et un deuxième état où elle est configurée pour s'engager avec l'implant afin de fournir une liaison entre l'implant (1) et le dispositif (20), et où la partie d'extrémité flexible comprend une butée (28a, 28b) à une distance de l'extrémité libre qui limite l'insertion de la partie d'extrémité flexible (21) dans l'implant à travers l'ouverture allongée, **caractérisé en ce que**, la partie d'extrémité flexible (21) comprend une saillie vers l'extérieur (25a, 25b) au niveau de son extrémité libre et une saillie vers l'extérieur (28a, 28b) à une distance de son extrémité libre, la distance étant supérieure à l'épaisseur de paroi autour de l'ouverture allongée (8) de sorte que l'implant (1) soit maintenu par le dispositif (20) dans le deuxième état.

2. Dispositif de la revendication 1, dans lequel la partie d'extrémité flexible (21) peut être retirée à travers l'ouverture (8) dans le premier état.

3. Dispositif de la revendication 1 ou 2, dans lequel les saillies vers l'extérieur s'étendent dans une direction de la hauteur de l'ouverture allongée (8).

4. Dispositif de l'une des revendications 1 à 3, dans lequel la partie d'extrémité flexible (21) comprend au moins deux languettes (23a, 23b) séparées par au moins une fente (24).

5. Dispositif de l'une des revendications 1 à 4, dans lequel la partie d'extrémité flexible (21) a une partie de surface chanfreinée (26a, 26b) au niveau de son extrémité libre qui est configurée pour permettre un coulissement le long d'un bord (8a, 8b) de l'ouverture allongée (8).

6. Dispositif de l'une des revendications 1 à 5, dans lequel la partie d'extrémité flexible (21) a une partie de surface arrière chanfreinée (27a, 27b) opposée à l'extrémité libre et configurée pour permettre un coulissement le long d'un bord (8a, 8b) de l'ouverture allongée (8).

7. Dispositif de l'une des revendications 1 à 6, dans lequel un élément d'écartement est agencé en coulissement dans la partie d'extrémité flexible (21).

8. Dispositif de la revendication 7, dans lequel l'élément d'écartement est une broche (40) qui peut adopter une première position rétractée dans laquelle la partie d'extrémité flexible (21) est configuré pour être compressible et extensible et une deuxième position en saillie dans laquelle elle fait saillie dans la partie d'extrémité flexible (21) empêchant ainsi la partie d'extrémité flexible d'être comprimée.

9. Dispositif de l'une des revendications 1 à 8, dans lequel la butée (28a, 28b) a une forme incurvée adaptée à une partie incurvée de la paroi latérale (4) de l'implant (1) autour de l'ouverture allongée (8).

10. Dispositif de l'une des revendications 6 à 9, dans lequel la partie d'extrémité flexible (21) et l'élément d'écartement sont reliés chacun à une partie de préhension (51, 52) d'une poignée (50).

11. Dispositif de la revendication 10, dans lequel la poignée (50) est configurée pour déplacer l'élément d'écartement par rapport à la partie d'extrémité flexible (21).

12. Système comprenant un implant intervertébral (1) et un dispositif d'insertion (20) de l'implant intervertébral dans un corps, où l'implant intervertébral (1) a une surface supérieure (2) configurée pour s'engager avec un premier corps vertébral, une surface inférieure (3) configurée pour s'engager avec un deuxième corps vertébral et une paroi latérale (4) reliant la surface supérieure (5) et la surface inférieure (3) et une ouverture allongée (8) dans la paroi latérale (4),
et où le dispositif d'insertion (20) est un dispositif selon l'une des revendications 1 à 11.

13. Système de la revendication 12, dans lequel l'ouverture allongée (8) a une forme arquée dans la direction de la longueur et où la partie d'extrémité flexible (21) a une partie arquée (25a, 25b ; 28a, 28b) adaptée à la forme arquée de l'ouverture (8).

14. Procédé pour coupler un implant intervertébral (1) à un dispositif d'insertion (20) de l'implant intervertébral (1), où l'implant intervertébral a une surface supérieure (2) configurée pour s'engager avec un premier corps vertébral, une surface inférieure (3) configurée pour s'engager avec un deuxième corps vertébral et une paroi latérale (4) reliant la surface supérieure (5) et la surface inférieure (3) et une ouverture allongée (8) dans la paroi latérale (4), l'ouverture ayant une hauteur et une longueur supérieure à la hauteur,
et où le dispositif d'insertion (20) est un dispositif selon l'une des revendications 1 à 11, le procédé comprenant une étape qui consiste
à introduire la partie d'extrémité flexible (21) dans l'ouverture (8) en la comprimant dans la direction de la hauteur de l'ouverture et à relier l'implant (1) au dispositif d'insertion (20) en écartant la partie d'extrémité flexible (21) dans la direction de la hauteur.

15. Procédé de la revendication 14, comprenant en outre une étape qui consiste à guider le dispositif d'insertion (20) le long de l'ouverture allongée (8) dans une direction de la longueur.
